# EUROPEAN PATENT APPLICATION

(11) **EP 1 491 626 A1**
(43) Date of publication of application: **29.12.2004**
(21) Application number: 03715583.5
(22) Date of filing: 28.03.2003
(51) Int. Cl.: C12N 15/00, C12Q 1/68, G01N 33/50

(54) **METHOD OF ASSAYING TARGET NUCLEIC ACID AND KIT THEREFOR**

(30) Priority: 29.03.2002 JP 2002097407
(71) Applicant: FUJIREBIO INC., Tokyo 103-0007 (JP)
(72) Inventor: HOTTA, Yoshiyuki c/o Fujirebio Inc., Chuo-ku, Tokyo 103-0007 (JP); HASEGAWA, Ayako c/o Fujirebio Inc., Chuo-ku, Tokyo §103-0007 (JP); ITO, Satoru c/o Fujirebio Inc., Chuo-ku, Tokyo 103-0007 (JP)
(74) Representative: Denison, Christopher Marcus
(86) International application number: PCT/JP2003/003953
(87) International publication number: WO 2003/083106

(57) **Abstract**

A method for measuring a target nucleic acid, which does not require skill and which enables to detect a SNP and to quantify the target nucleic acid simply in a short time is disclosed. In the method of the present invention, the target nucleic acid, a labeled probe which is a labeled nucleic acid which hybridizes with the target nucleic acid, a non-labeled probe which is a nucleic acid having a nucleotide sequence complementary to a region in the target nucleic acid, which region is different from the region with which the labeled probe hybridizes, and an immobilized probe which is a nucleic acid bound to a support, which nucleic acid has a nucleotide sequence complementary to a region in the target nucleic acid, which region is different from the region with which the labeled probe hybridizes are reacted; and the label of the labeled probe bound to the support is measured. An end region of the region with which the non-labeled probe hybridizes overlaps with an end region of the region with which the immobilized probe hybridizes.

SEQUENCE LISTING
<110> FUJIREBIO INC.
<120> Method for measuring nucleic acids and kit therefor
<130> 03PF260-PCT
<160> 28

<210> 1
<211> 21
<212> DNA
<213> Artificial Sequence
<220>
<223> oligonucleotide probe
<400> 1
ttgtggcttg gctgcaccca g 21

<210> 2
<211> 20
<212> DNA
<213> Artificial Sequence
<220>
<223> oligonucleotide probe
<400> 2
ggccagacag cggcgccatg 20

<210> 3
<211> 20
<212> DNA

<213>  Artificial Sequence

<220>

<223>  oligonucleotide probe

<400>  3

cggccagaca gcggcgccat                                                    20

<210>  4

<211>  20

<212>  DNA

<213>  Artificial Sequence

<220>

<223>  oligonucleotide probe

<400>  4

gcggccagac agcggcgcca                                                    20

<210>  5

<211>  20

<212>  DNA

<213>  Artificial Sequence

<220>

<223>  oligonucleotide probe

<400>  5

tgcggccaga cagcggcgcc                                                    20

<210>  6

<211>  20

<212>  DNA

<213>  Artificial Sequence

<220>

<223>  oligonucleotide probe

<400>  6

gtgcggccag acagcggcgc                                                    20

<210>  7

<211>  20

<212>  DNA

<213>  Artificial Sequence

<220>

<223>  oligonucleotide probe

<400>  7

cagcagctga aatagcagtg                                                    20

<210>  8

<211>  21

<212>  DNA

<213>  Artificial Sequence

<220>

<223>  oligonucleotide probe

<400>  8

ttgtggcttg gctgcaccca g                                                  21

<210>  9

<211>  20

<212>  DNA

<213>  Artificial Sequence

<220>

<223>  oligonucleotide probe

<400>  9

gcggccagac agcggcgcca                                                    20

<210>  10

<211>  20

<212>  DNA

<213>  Artificial Sequence

<220>

<223>  oligonucleotide probe

<400>  10

agcagctgaa atagccgtgc                                                    20

<210>  11

<211>  20

<212>  DNA

<213>  Artificial Sequence

<220>

<223>  oligonucleotide probe

<400>  11

atcttcacgc cctacggctg                                                    20

<210>  12

<211>  20

<212>  DNA

<213>  Artificial Sequence

<220>

<223>  oligonucleotide probe

<400>  12

tggctggagc cgtggtccgg                                                    20


<210>  13

<211>  25

<212>  DNA

<213>  Artificial Sequence

<220>

<223>  oligonucleotide probe

<400>  13

ggtggcctgg gcggtgtcca actgg                                              25


<210>  14

<211>  21

<212>  DNA

<213>  Artificial Sequence

<220>

<223>  oligonucleotide probe

<400>  14

gcaggccttg cagaaatcca g                                                  21


<210>  15

<211>  20

<212>  DNA

<213>  Artificial Sequence

<220>

<223>  oligonucleotide probe

<400>  15

ctgcgcaccg tctggtacct                                                    20

<210>  16

<211>  20

<212>  DNA

<213>  Artificial Sequence

<220>

<223>  oligonucleotide probe

<400>  16

tgaaccaagc cgctttgctg                                                    20

<210>  17

<211>  20

<212>  DNA

<213>  Artificial Sequence

<220>

<223>  oligonucleotide primer used for PCR

<400>  17

atggatcccc tgggtgcagc                                                    20

<210>  18

<211>  20

<212>  DNA

<213>  Artificial Sequence

<220>

<223>  oligonucleotide primer used for PCR

<400>  18

agcaggatca ggagggtggg                                                    20

<210>  19

<211>  25

<212>  DNA

<213>  Artificial Sequence

<220>

<223>  oligonucleotide primer used for PCR

<400>  19

acttggagcc accccctaac tgcca                                              25

<210>  20

<211>  25

<212>  DNA

<213>  Artificial Sequence

<220>

<223>  oligonucleotide primer used for PCR

<400>  20

tgagtccggc ttccagttgg acacc                                              25

<210>  21

<211>  20

<212>  DNA

<213>  Artificial Sequence

<220>

<223>  oligonucleotide primer used for PCR

<400>  21
tccagagccc caaggacctg                                                    20


<210>  22

<211>  19

<212>  DNA

<213>  Artificial Sequence

<220>

<223>  oligonucleotide primer used for PCR

<400>  22
tcaggtgaac caagccgct                                                     19


<210>  23

<211>  200

<212>  DNA

<213>  Homo sapiens

<400>  23
atggatcccc tgggtgcagc caagccacaa tggccatggc gccgctgtct ggccgcactg    60

ctatttcagc tgctggtggc tgtgtgtttc ttctcctacc tgcgtgtgtc ccgagacgat   120

gccactggat cccctagggc tcccagtggg tcctcccgac aggacaccac tcccacccgc   180

cccaccctcc tgatcctgct                                                200


<210>  24

<211>  200

<212>  DNA
<213>  Homo sapiens
<400>  24
atggatcccc tgggtgcagc caagccacaa tggccatggc gccgctgtct ggccgcacgg    60
ctatttcagc tgctggtggc tgtgtgtttc ttctcctacc tgcgtgtgtc ccgagacgat   120
gccactggat cccctagggc tcccagtggg tcctcccgac aggacaccac tcccacccgc   180
cccaccctcc tgatcctgct                                               200

<210>  25
<211>  206
<212>  DNA
<213>  Homo sapiens
<400>  25
acttggagcc accccctaac tgccagcacc tggaagccct ggacagatac ttcaatctca    60
ccatgtccta ccgcagcgac tccgacatct tcacgcccta cggctggctg gagccgtggt   120
ccggccagcc tgcccaccca ccgctcaacc tctcggccaa gaccgagctg gtggcctggg   180
cggtgtccaa ctggaagccg gactca                                        206

<210>  26
<211>  206
<212>  DNA
<213>  Homo sapiens
<400>  26
acttggagcc accccctaac tgccagcacc tggaagccct ggacagatac ttcaatctca    60
ccatgtccta ccgcagcgac tccgacatct tcacgcccta cagctggctg gagccgtggt   120
ccggccagcc tgcccaccca ccgctcaacc tctcggccaa gaccgagctg gtggcctggg   180
cggtgtccaa ctggaagccg gactca                                        206

<210>  27

<211>  200

<212>  DNA

<213>  Homo sapiens

<400>  27

tccagagccc caaggacctg gcccggtacc tgcaggagct ggacaaggac cacgcccgct    60

acctgagcta ctttcgctgg cgggagacgc tgcggcctcg ctccttcagc tgggcactgg   120

atttctgcaa ggcctgctgg aaactgcagc aggaatccag gtaccagacg gtgcgcagca   180

tagcggcttg gttcacctga                                                200

<210>  28

<211>  200

<212>  DNA

<213>  Homo sapiens

<400>  28

tccagagccc caaggacctg gcccggtacc tgcaggagct ggacaaggac cacgcccgct    60

acctgagcta ctttcgctgg cgggagacgc tgcggcctcg ctccttcagc tgggcactgg   120

atttctgcaa ggcctgctgg aaactgcagc aggaatccag gtaccagacg gtgcgcagca   180

aagcggcttg gttcacctga                                                200

## Description

### Technical Field

The present invention relates to a method for measuring a target nucleic acid and to a kit therefor. The method of the present invention is useful for detections of single nucleotide polymorphisms (SNPs) and for judgments of zygosities of genes.

### Background Art

Judgments of mutations of nucleic acids are carried out by cleaving an isolated nucleic acid with a restriction enzyme, and detecting the nucleic acid fragments on a gel (such as agarose or acrylamide) by electrophoresis. However, cleaving of the nucleic acid by the restriction enzyme is time-consuming, and electrophoresis is laborious. Further, carcinogenic ethylene bromide is used, so that special care is required when discarding it.

SNPs are also detected by DNA sequencing or by using DNA chips. However, these techniques have drawbacks in that they require skill and are expensive.

Cytometry 39: 131-140 (2000) discloses a method comprising hybridizing a target nucleic acid, a probe labeled with a fluorescent dye, which is complementary to the target nucleic acid, and a probe having a region complementary to the region of the target nucleic acid, which region of the probe is adjacent to the labeled probe, and a region complementary to a probe immobilized on a particle; carrying out ligation by ligase; hybridizing the resultant with the probe immobilized on the particle; and measuring the fluorescence intensity of the labeled probe bound to the particle by a flow cytometer. If the sequence adjacent to the two probes has a mutant sequence, the ligation does not occur, so that the labeled probe is not bound to the particle, and the fluorescence is not observed. The target nucleic acid is detected utilizing the difference. This measuring method has a drawback in that it is time-consuming because the hybridization operation is carried out again after the ligation operation.

### Disclosure of the Invention

Accordingly, an object of the present invention is to provide a method for measuring a target nucleic acid by which detections of SNPs or quantifications of target nucleic acids may be attained without skill, simply in a short time.

The present inventors intensively studied to discover, surprisingly, that by employing a method for measuring a target nucleic acid by reacting the target nucleic acid, an immobilized probe which hybridizes with the target nucleic acid, and a labeled probe; and by measuring the label bound to the support, and by further reacting a non-labeled probe which hybridizes with a region whose end region overlaps with the end region of the region with which the immobilized probe hybridizes, the specificity of the reaction between the immobilized probe and the target nucleic acid is increased so that even the difference of one base can be distinguished, thereby completing the present invention.

That is, the present invention provides a method for measuring a target nucleic acid comprising reacting the target nucleic acid, a labeled probe which is a labeled nucleic acid which hybridizes with the target nucleic acid, a non-labeled probe which is a nucleic acid having a nucleotide sequence complementary to a region in the target nucleic acid, which region is different from the region with which the labeled probe hybridizes, and an immobilized probe which is a nucleic acid bound to a support, which nucleic acid has a nucleotide sequence complementary to a region in the target nucleic acid, which region is different from the region with which the labeled probe hybridizes; and measuring the label of the labeled probe bound to the support; wherein an end region of the region with which the non-labeled probe hybridizes overlaps with an end region of the region with which the immobilized probe hybridizes. The present invention also provides a method for detecting a single nucleotide polymorphism, comprising applying the method according to the present invention to a test sample containing the target nucleic acid or a mutant nucleic acid having a single nucleotide polymorphism of the target nucleic acid; and judging from the obtained measurement result whether the nucleic acid in the test sample is the target nucleic acid or the mutant nucleic acid. The present invention further provides a method for judging zygosity of a gene, comprising applying the method according to the present invention to a test sample containing a gene whose allele comprises a normal gene and an abnormal gene having a single nucleotide polymorphism; and judging from the obtained measurement result the zygosity of the gene. The present invention still further provides a kit for measuring a nucleic acid, which is used for the method according to the present invention, comprising at least the labeled probe, the non-labeled probe and the immobilized probe.

By the present invention, a method for measuring a target nucleic acid by which detections of SNPs and quantifications of nucleic acids may be attained without skill, simply in a short time was provided. By the method of the present invention, difference by only one base can be distinguished, and zygosity of an allele having a difference by only one base can be judged.

### Brief Description of the Drawings

Fig. 1 is a schematic view for explaining the method of the present invention.
Fig. 2 is a drawing showing the nucleotide sequences of the target nucleic acids and various probes used in Examples of the present invention, as well as the regions at which they hybridize.
Fig. 3 is a graph showing the change of the fluorescent intensity depending on the position of the non-labeled probe (the numbers along the abscissa indicate the positions of the non-labeled probes, corresponding to the nucleotide sequence of FUT III DNA, and the numbers in the parentheses indicate the number of bases at which the 5'-end region of the non-labeled probe and the 3'-end region of the immobilized probe overlap.
Fig. 4 is a graph showing the change of the fluorescence intensity with time depending on the existence of the added non-labeled probe.
Fig. 5 is a graph showing the differences in the fluorescence intensities depending on the gene type (homozygote, heterozygote) of the target nucleic acid.

### Best Mode for Carrying Out the Invention

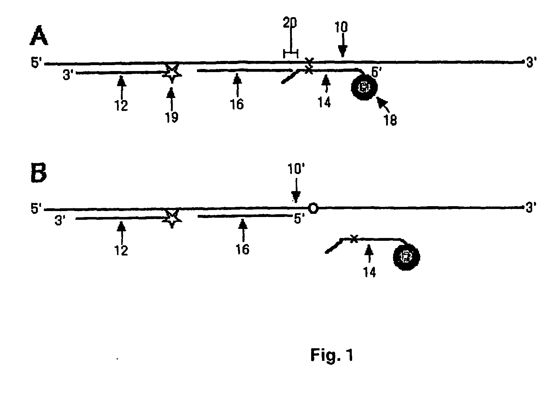

An example of the method of the present invention will now be described based on the schematic drawing shown in Fig. 1A. As described above, in the method of the present invention, a target nucleic acid 10, a labeled probe 12, an immobilized probe 14 and a non-labeled probe 16 are annealed, thereby hybridizing the target nucleic acid 10 with the labeled probe 12, the immobilized probe 14 and with the non-labeled probe 16, respectively. The target nucleic acid 10 in a test sample is then measured by measuring the label 19 of the labeled probe 12 bound to the support 18 via the target nucleic acid 10, on which support the immobilized probe 14 is immobilized,.

Each constituent will now be described in detail. The target nucleic acid 10 is a nucleic acid to be measured, and is not restricted at all. Examples of the target nucleic acid include genomic genes of human and other organisms, cDNAs, and genes of pathogenic microorganisms and viruses, but the target nucleic acid is not restricted thereto. Since the method of the present invention has a high specificity by which the difference by even one base can be distinguished and is applicable to the detections of SNPs, it is especially useful for the cases wherein the target nucleic acid is one in which a SNP is to be detected. The target nucleic acid may be either a DNA or RNA, or may be an artificial nucleic acid which can hybridize with DNA or RNA. The target nucleic acid may be one which was amplified by a nucleic acid-amplification method such as PCR, or may be a nucleic acid which was not amplified. In the present description, the term "measure" includes both detection and quantification. The size of the target nucleic acid is not restricted, and is usually not less than 40 bases, preferably not less than 60 bases because it has to hybridize with three types of probes. Although there is no upper limit of the size of the target nucleic acid, it is usually preferred that the upper limit is about 100 to 500 bases.

The labeled probe 12 is a labeled nucleic acid which hybridizes with a region in the target nucleic acid. Although the labeled probe preferably has a nucleotide sequence complementary to the nucleotide sequence of the region (hereinafter also referred to as "labeled probe-hybridizing region") in the target nucleic acid, with which the labeled probe is to hybridize, it may contain non-complementary nucleotides usually in the number of not more than 10%, preferably not more than 5%, as long as it can hybridize with the target nucleic acid under the hybridization conditions under which the hybridizations of the non-labeled probe and the immobilized probe with the target nucleic acid are attained. Although the size of the labeled probe is not restricted and may be any size which is effective for the detection of the target nucleic acid, the size is preferably not less than 15 bases, and especially preferably about 20 to 50 bases. The label of the labeled probe may be any label by which the probe can be measured, and any of the labels used for the conventional labeled probes may be employed. That is, examples of the labels include fluorescent labels, radioactive labels, enzyme labels and biotin labels. Among these, fluorescent labels are preferred in view of the facts that they can be measured safely without using an expensive apparatus, and that they do not interfere with the hybridization with the target nucleic acid. The label may be bound to any part of the probe, and the binding may be attained by a well-known method. The probe may be either a DNA or RNA, or may be an artificial nucleic acid which can hybridize with DNA or RNA.

The immobilized probe 14 is a nucleic acid having a nucleotide sequence complementary to that of a region (hereinafter also referred to as "immobilized probe-hybridizing region") in the target nucleic acid 10, which region is different from the above-mentioned labeled probe-hybridizing region, which probe is immobilized on a support. The size of the immobilized probe is not restricted and may be any size which is effective for the detection of the target nucleic acid, the size is preferably not less than 15 bases, and especially preferably about 20 to 50 bases. As the support, anything which can immobilize nucleic acids may be employed. Examples of the support include particles such as polystyrene particles, glass particles and latex particles; magnetic particles which are the above-mentioned particles in which ferrite is admixed to give the particles magnetic force; and inner walls of wells of microplates. The support is preferably bound to an end of the nucleic acid so that the hybridization between the immobilized probe and the target nucleic acid is not interfered. Immobilization of nucleic acids to the support can be carried out by a well-known method. Polystyrene particles or the like having functional groups such as carboxyl groups bound to the surface thereof so as to enable the support to easily bind the nucleic acids are commercially available, and such commercially available particles for immobilization of nucleic acids may preferably be employed. The nucleic acid of the immobilized probe 14 may be directly bound to the support 18, or may be bound to the support via a spacer region having a nucleotide sequence not involved in the hybridization with the target nucleic acid. Thus, the cases where a nucleic acid having the sequence complementary to the immobilized probe-hybridizing region is indirectly bound to the support via the spacer region are also included in the cases where "a nucleic acid having a complementary nucleotide sequence is bound to a support" as the term is used in the present invention.

The non-labeled probe 16 is a nucleic acid having a nucleotide sequence complementary to a region in the target nucleic acid 10, which region is different from the labeled probe-hybridizing region. The size of the non-labeled probe is not restricted and may be any size which is effective for the detection of the target nucleic acid. The size is preferably not less than 15 bases, and especially preferably about 20 to 50 bases.

An end region of the non-labeled probe-hybridizing region and an end region of the immobilized probe-hybridizing region overlap (this overlapping region is also hereinafter referred to as "overlapping region"). The overlapping region is denoted by reference numeral 20 in Fig. 1A. The size of the overlapping region is not restricted, and preferably 1 to 5 bases, more preferably 1 to 3 bases. By providing such an overlapping region 20, the specificity of the reaction between the immobilized probe 14 and the target nucleic acid 10 is increased, so that the difference by only one base can be detected.

The method of the present invention has an excellent effect that it can measure the target nucleic acid specifically distinguishing a single nucleotide mutation in cases where there is a mutated type of the target nucleic acid, particularly a SNP. In cases where the target nucleic acid is to be measured specifically distinguishing such a mutation, the immobilized probe is so designed that the site of mutation is located in the immobilized probe-hybridizing region. The site of mutation is preferably apart from the overlapping region 20 by one base (i.e., adjacent to an end of the overlapping region) to 5 bases. In Fig. 1A, the site of mutation on the target nucleic acid 10 is denoted by Y. The base in the immobilized probe 14, which pairs with Y is denoted by X. In the present description, the nucleic acid mutated from the target nucleic acid is called a mutated type, and the mutated type is not necessarily an abnormal type. In cases where an abnormal type is employed as the target nucleic acid for the purpose of detecting the abnormal type gene, the normal type is the mutated type.

In the example shown in Fig. 1 A, the 5'-end region of the immobilized probe-hybridizing region and the 3'-end region of the non-labeled probe-hybridizing region overlap. However, these probes may also be designed inversely to this, such that the 3'-end region of the immobilized probe-hybridizing region and the 5'-end region of the non-labeled probe-hybridizing region overlap (in this case, however, the 3'-end of the immobilized probe is bound to the support). The labeled probe-hybridizing region may be located at an arbitrary region which does not overlap with the non-labeled probe-hybridizing region and the immobilized probe-hybridizing region.

As described above, in the method of the present invention, the target nucleic acid 10, the labeled probe 12, the immobilized probe 14 and the non-labeled probe 16 are hybridized. The hybridization reaction may be carried out such that the four types of the nucleic acids are reacted simultaneously, or the target nucleic acid 10 and arbitrary probes are sequentially reacted, or the target nucleic acid 10 is hybridized with arbitrary two types of probes, and the third probe is then reacted. The reaction conditions are not restricted, and may appropriately be selected depending on the sizes of the probes and the like. Under the conditions where the target nucleic acid 10, the immobilized probe 14 and the non-labeled probe 16 co-exist, the hybridization reaction may usually be carried out at 30°C to 60°C for not less than 10 minutes, preferably at 40°C to 50°C for about 10 minutes to 60 minutes. Under the conditions where one of the immobilized probe 14 and the non-labeled probe 12 does not exist, the hybridization reaction may be carried out in the same manner as described above, or may be carried out while cooling the reaction mixture from a high temperature to a low temperature. For example, in the Example below, the target nucleic acid 10, the labeled probe 12 and the non-labeled probe 16 are first reacted, and then the immobilized probe 14 is reacted. In this case, the reaction among the target nucleic acid 10, the labeled probe 12 and the non-labeled probe 16 is carried out while cooling the reaction solution on ice from the denaturation temperature (95°C). By cooling the reaction solution from a high temperature to a low temperature, the temperature at which the hybridization occurs is inevitably passed, so that the target nucleic acid and the probes can be hybridized.

After the hybridization reaction, the label 19 bound to the support 18 is measured. The measurement of the label 19 may be carried out by a well-known method suited for the label. In cases where the support 18 is a particle, the label is measured after collecting the supports 18 by centrifugation or by applying magnetic force (when the supports are magnetic particles). In cases where the target nucleic acid 10 exists in the test sample, the labeled probe 12 is bound to the support 18 via the target nucleic acid 10 and the nucleic acid portion of the immobilized probe 14. In cases where the target nucleic acid 10 does not exist, the labeled probe 12 is not bound to the support 18. Therefore, by measuring the label 19 bound to the support 18, the target nucleic acid 10 in the test sample can be detected. Further, the more the amount of the label 19 bound to the support 19, the more the amount of the target nucleic acid 10 in the test sample. Therefore, the target nucleic acid 10 may also be quantified by quantifying the label 19.

By applying the above-described method of the present invention to a test sample containing the target nucleic acid or a mutated nucleic acid having a single nucleotide polymorphism of the target nucleic acid, it can be judged from the measurement results whether the nucleic acid contained in the test sample is the target nucleic acid or the mutated nucleic acid. This will now be described referring to Fig. 1B. In Fig. 1B, 10' denotes a mutated nucleic acid wherein the single base Y in the target nucleic acid 10 is mutated, and the mutated base is denoted by ○. The mutated nucleic acid 10' in which a single base is mutated and the immobilized probe 14 do not hybridize as shown in Fig. 1B, or even if they hybridize, the number of the immobilized probes which hybridize with the mutated nucleic acid is smaller than the number of the immobilized probes which hybridize with the target nucleic acid to a distinguishable extent. Therefore, the amount of the label bound to the support 18 is smaller to a distinguishable extent, so that it can be judged whether the nucleic acid contained in the test sample is the target nucleic acid or the mutated nucleic acid.

Further, by applying the above-described method of the present invention to a test sample containing a gene whose allele comprises a normal gene and an abnormal gene having a single nucleotide polymorphism, the zygosity of the gene in the test sample can be judged from the measurement results. Thus, by the method of the present invention, it can be judged whether the gene of interest is homozygote or heterozygote. When the target nucleic acid is in homozygote, the measured amount of the label is the largest, when the target nucleic acid and the mutated nucleic acid are in heterozygote, the measured amount of the label is the second largest, and when the mutated type is in homozygote, the measured amount of the label is the smallest. Therefore, by measuring the amount of the label bound to the support according to the present invention, the zygosity of the allele can be judged.

The present invention also provides a kit for measuring a nucleic acid used for the above-described method of the present invention, which comprises at least the above-described labeled probe, the above-described non-labeled probe and the above-described immobilized probe. These constituents are as described above. The kit may further contain a buffer or the like used for the reactions.

### Examples

The present invention will now be described concretely by way of examples.

### Fucose Transferase 3 (FUTIII) Gene as Target Nucleic Acid

FUTIII gene is a gene involved in the transfer of fucose to terminals of sugar chains to synthesize blood type antigens and cancer-associated antigens (CA19-9), and four types of the gene, that is, wild type (WT), and mutated types (Le1, Le2 and Le3) are known. As the sites of mutation, the 59th, 508th and 1067th bases are known (Cancer Res. 58, 512-518(1998); "Protein, Nucleic Acid and Enzyme", Vol.43 No.16 (1998)).

### 1. Preparation of Target Nucleic Acids

Using each of the FUTIII genes as a template, each nucleic acid was amplified by polymerase chain reaction (PCR) method (30 cycles of 94°C for 1 minute, 55°C for 1 minute and 72°C for 1 minute), and each amplification product was purified by using MO BIO : ULTRA CLEAN DNA Purification kit: Catalog #12100-300, followed by measurement of the concentration of each amplification product by measuring the absorbance at 260 nm. The combinations of the primers used for the nucleic acids including the sites of mutation were as follows: As for the nucleic acid (WT<59> or Le<59>) for which the site of mutation at the 59th base was targeted, the region from 1st to 200th bases (200 bp) was amplified using a sense primer (atggatcccctgggtgcagc: 20mer) located at 1st to 20th bases and an anti-sense primer (agcaggatcaggagggtggg: 20mer) corresponding to 180th to 200th bases. As for the nucleic acid (WT<508> or Le<508>) for which the site of mutation at the 508th base was targeted, the region from 407th to 612th bases (206 bp) was amplified using a sense primer (acttggagccaccccctaactgcca: 25mer) located at 407th to 431 st bases and an anti-sense primer (tgagtccggcttccagttggacacca: 25mer) corresponding to 588th to 612th bases. As for the nucleic acid (WT<1067> or Le<1067>) for which the site of mutation at the 1067th base was targeted, the region from 887th to 1086th bases (200 bp) was amplified using a sense primer (tccagagccccaaggacctg: 20mer) located at 887th to 906th bases and an anti-sense primer (tcaggtgaaccaagccgct: 19mer) located at 887th to 1086th bases.

### 2. Preparation of Immobilized Probes

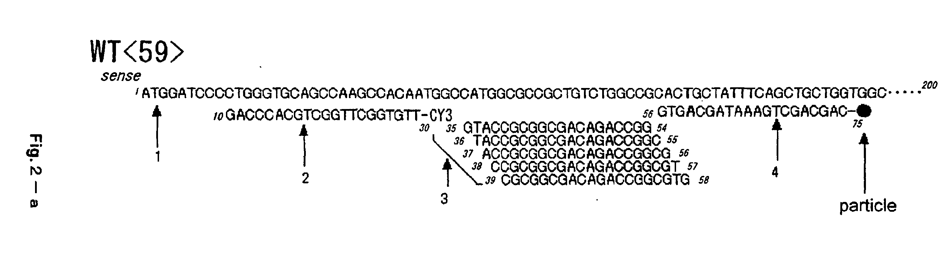

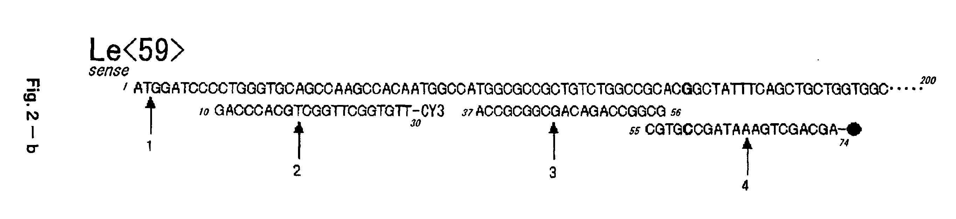

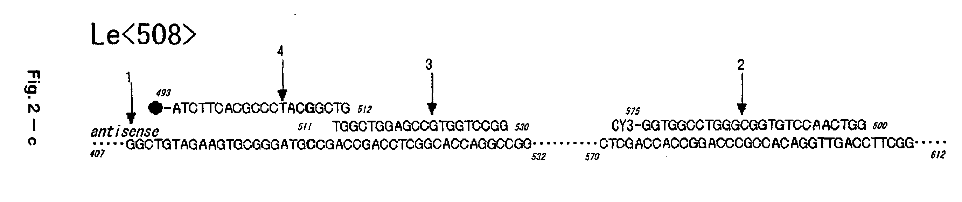

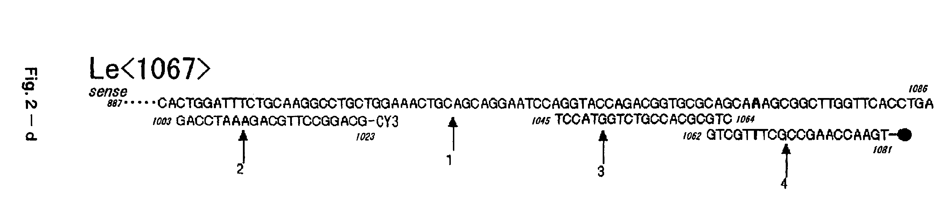

Probes for immobilization (the nucleotide sequences 4 shown in Fig. 2-a, -b, -c and -d: 20 mer, SEQ ID NOs: 7, 10, 11 and 16) (Amersham Bioscience) were designed such that they had nucleotide sequences complementary to those of FUTIII genes and that the site of mutation was located within the region from the 4th to 6th bases from the 3'-end of the probes, and an amino group was bound to the 5'-end thereof, and each probe was immobilized on a particle (immobilizing particle).

The particles used were polystyrene particles having a diameter of 5.5 µm and having carboxyl groups on the surfaces thereof (Bangs Laboratories, Inc. Catalog Code : PC06N, Polymer Description : P(S/5.5%DVB/5%MMA)).

Immobilization of the probes for immobilization on the particles was carried out by suspending about 10⁸ particles in 25 µl of 0.1M MES (2-[N-morpholino]ethanesulfonic acid) (pH4.5); adding 5 µl of the probe for immobilization (about 100 pmol/µl) and 1.25 µl of EDC (1-ethyl-3-(dimethylaminopropyl)carbodiimide hydrochloric acid salt) (10 mg/ml); and allowing the mixture to react at 37°C for 1 hour (condensation reaction between the carboxyl groups on the particles and the amino groups of the probes for immobilization), thereby preparing immobilizing particles. The obtained particles were washed with 0.02% Tween 20 (trademark) and then with 0.1% SDS solution, and the medium was replaced with 0.1M MES (pH4.5), followed by storage at 4°C.

### 3. Preparation of Non-labeled Probes

In the hybridization between the sample nucleic acid and the immobilized probe, the position of the non-labeled probe at the 3'-side of the immobilized probe will now be described. Probes (about 20 mer) (Amersham Bioscience) including a probe of which 5'-end was apart from the 3'-end of the immobilized probe by one base, to a probe of which 5'-end overlapped with the 3'-end of the immobilized probe by three bases, were prepared (the nucleotide sequences 3 shown in Fig. 2-a, -b, -c and -d, SEQ ID NOs: 2-6, 9, 12 and 15).

### 4. Preparation of Labeled Probes

Probes (Amersham Bioscience) having nucleotide sequences which did not overlap with those of the immobilized probes and the non-labeled probes were prepared, whose 5'-ends were labeled with a fluorescent dye (CY3), were prepared (the nucleotide sequences 2 shown in Figs. 2-a, -b, -c and -d, SEQ ID NOs: 1, 8, 13 and 14).

### 5. Method for Measurement

An aqueous solution (total volume of 10 µl) containing a sample nucleic acid (800 fmol), non-labeled probe (50 pmol) and a labeled probe (1 pmol) was subjected to thermal denaturation (95°C for 5 minutes), and the solution was cooled on ice (1 minute). To this solution, immobilizing particles (about 100,000 particles suspended in 10 µl of 10 x SSC, final concentration: 5 x SSC) were added and hybridization with the target nucleic acid was allowed (40°C for 2 hours, in the analyses with time, 0 to 2 hours), followed by centrifugation (15,000 rpm for 2 minutes), thereby removing the reaction solution. The particles were then washed with 50 µl of TBS-Triton (10mM Tris-Cl, 0.2% NaCl, 0.1% NaN₂, 0.01% Triton X-100, pH7.2), and then centrifuged again, followed by suspending the particles in 200 µl of a buffer for measurement (P/N8599600, a sheath fluid specialized for flow cytometer, Beckman Coulter). Using a flow cytometer (EPICS-PROFILE II, Beckman Coulter), the fluorescent intensity on the particles were measured, and existence of the target nucleic acid was identified based on the fluorescent intensity.

### 6. Position of Non-labeled Probes

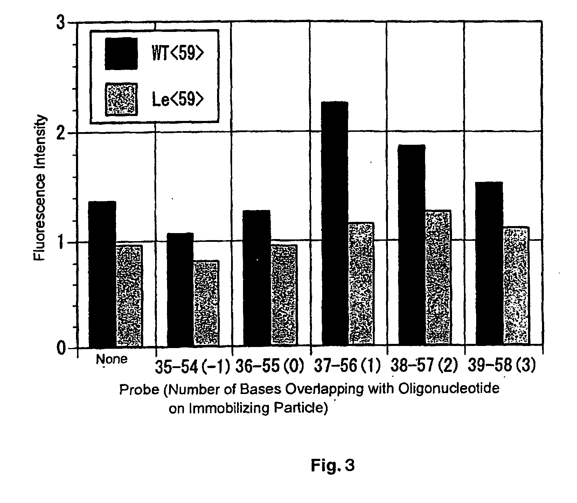

Employing the wild type of FUTIII gene (1 shown in Fig. 2-a, WT<59>; 1 st to 200th, 200 bp) as the target nucleic acid, detection of the target nucleic acid was tried using as the probe immobilized on the particles the antisense sequence (4 shown in Fig. 2-a) corresponding to 56th to 75th base in FUTIII gene, a non-labeled probe (3 shown in Fig. 2, SEQ ID NOs: 2-6) having a varying number of overlapping bases, the overlapping being the overlap between the 5'-end of the non-labeled probe and 3'-end of the immobilized probe, and a labeled probe (2 shown in Fig. 2-a). When the 3'-end region of the immobilized probe and the 5'-end region of the non-labeled probe overlapped by 1 to 3 bases, increase of the fluorescence intensity of the WT<59> which was the target nucleic acid was observed, while the fluorescence intensity of the mutated type (1 shown in Fig. 2-b: Le<59>; 1 st to 200th, 200 bp) did not substantially change. By this, it was shown that the specificity of the hybridization was increased by overlapping the 3'-end region of the immobilized probe and 5'-end region of the non-labeled probe by 1 to 3 bases (Fig. 3).

### 7. Effect by Non-labeled Probe

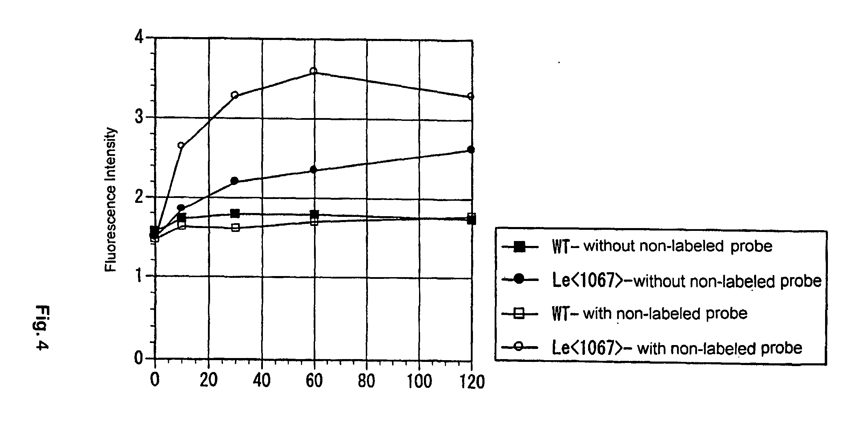

Employing the mutated type of FUTIII gene (Le<1067>; 887th to 1086th, 200 bp) as the target nucleic acid, studies were made using an immobilized probe (tgaaccaagccgctttgctg, SEQ ID NO: 16) which was the anti-sense chain corresponding to 1062nd to 1081 st base of FUTIII gene, a non-labeled probe (ctgcgcaccgtctggtacct, SEQ ID NO: 15) which was the anti-sense chain which overlapped with the 3'-end region of the immobilized probe by three bases, and a labeled probe (gcaggccttgcagaaatccag, SEQ ID NO: 14) (Fig. 2-d). By adding the non-labeled probe to the sample nucleic acid, sharp increase of the fluorescence intensity of the target nucleic acid with time was observed when compared with the sample to which the non-labeled probe was not added (Fig. 4). On the other hand, with the wild type nucleic acid (WT<1067>; 887th to 1086th, 200bp) which was not the target nucleic acid, change of the fluorescence intensity was not observed. It was shown that the non-labeled probe improves the efficiency of the specific hybridization between the target nucleic acid and the immobilizing particle.

### 8. Detection of Target Nucleic Acid

FUTIII gene includes homozygote and heterozygote of the gene types. Whether the zygosity of the target nucleic acid can be judged or not is described.

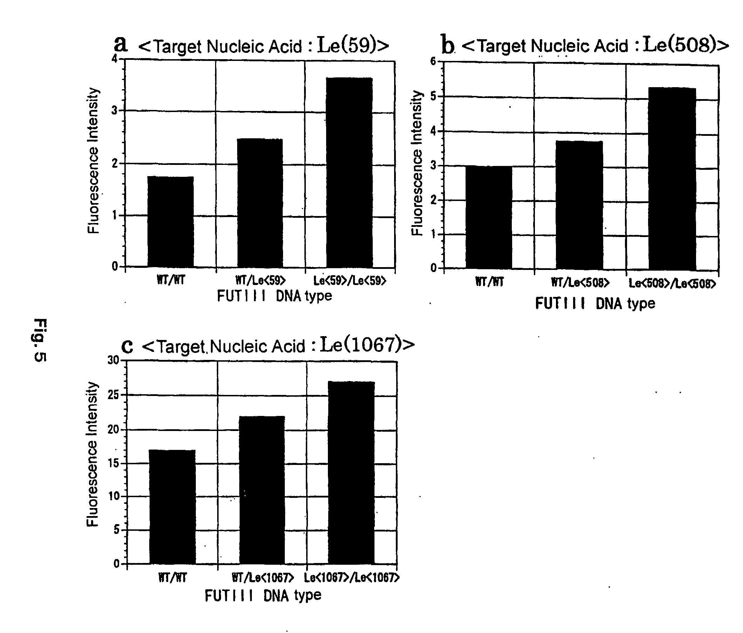

When the mutated nucleic acid having the mutation at the 59th base of FUTIII gene was employed as the target nucleic acid (1 shown in Fig. 2-b), the fluorescence intensity was (Le<59>/Le<59>) > (WT<59>/Le<59>) > (WT<59>/WT<59>) (Fig. 5-a). When the mutated nucleic acid having the mutation at the 508th base of FUTIII gene was employed as the target nucleic acid (1 shown in Fig. 2-c), the fluorescence intensity was (Le<508>/Le<508>) > (WT<508>/Le<508>) > (WT<508>/WT<508>) (Fig. 5-b). When the mutated nucleic acid having the mutation at the 1067th base of FUTIII gene was employed as the target nucleic acid (1 shown in Fig. 2-d), the fluorescence intensity was (Le<1067>/Le<1067>) > (WT<1067>/Le<1067>) > (WT<1067>/WT<1067>) (Fig. 5-c). (As for the nucleotide sequences of the labeled (CY3) probe, non-labeled probe and the immobilized probe, see Figs. 2-b, -c and -d).

The order of the fluorescence intensity from the larger to smaller was, in the order mentioned, homozygote of the target nucleic acid, heterozygote of the target nucleic acid and the nucleic acid which was not the target nucleic acid, and the homozygote of the nucleic acid which was not the target nucleic acid. Thus, existence of the target nucleic acid and the zygosity (homozygote or heterozygote) of the gene were able to be judged.

The meanings of the reference numerals in Figs. 2-a, -b and -c are summarized below. For example, 1 shown in Fig. 2-a is denoted "a-1".
a-1: target nucleic acid, wild type (WT(59))
a-2: CY3-labeled probe for detecting the WT(59) which was the target nucleic acid
a-3: non-labeled probe used for studying the change of the specificity of the hybridization between the nucleic acid (a-1) and the immobilized probe (a-4) depending on the design of the non-labeled probe.
a-4: immobilized probe for detecting WT(59)
b-1: target nucleic acid, mutated type (Le(59))
b-2: CY3-labeled probe for detecting Le(59) which was the target nucleic acid
b-3: non-labeled probe added for increasing the specificity of the hybridization between the target nucleic acid (b-1) and the immobilized probe (b-4)
b-4: immobilized probe for detecting Le(59)
c-1: target nucleic acid, mutated type (Le(508))
c-2: CY3-labeled probe for detecting Le(508) which was the target nucleic acid
c-3: non-labeled probe added for increasing the specificity of the hybridization between the target nucleic acid (c-1) and the immobilized probe (c-4)
c-4: immobilized probe for detecting Le(508)
d-1: target nucleic acid, mutated type (Le(1067))
d-2: CY3-labeled probe for detecting Le(1067) which was the target nucleic acid
d-3: non-labeled probe added for increasing the specificity of the hybridization between the target nucleic acid (d-1) and the immobilized probe (d-4)
d-4: immobilized probe for detecting Le(1067)

## Claims

1. A method for measuring a target nucleic acid comprising reacting said target nucleic acid, a labeled probe which is a labeled nucleic acid which hybridizes with said target nucleic acid, a non-labeled probe which is a nucleic acid having a nucleotide sequence complementary to a region in said target nucleic acid, which region is different from the region with which said labeled probe hybridizes, and an immobilized probe which is a nucleic acid bound to a support, which nucleic acid has a nucleotide sequence complementary to a region in said target nucleic acid, which region is different from the region with which said labeled probe hybridizes; and measuring the label of said labeled probe bound to said support; wherein an end region of said region with which said non-labeled probe hybridizes overlaps with an end region of said region with which said immobilized probe hybridizes.

2. The method according to claim 1, wherein the number of bases in the overlapping region is 1 to 5.

3. The method according to claim 2, wherein the number of bases in the overlapping region is 1 to 3.

4. The method according to any one of claims 1 to 3, wherein the region with which the 5'-end region of said non-labeled probe hybridizes and the region with which the 3'-end region of said immobilized probe hybridizes overlap.

5. The method according to any one of claims 1 to 4, wherein a mutant of said target nucleic acid exists, and the site of mutation is located within said region with which said immobilized probe hybridizes.

6. The method according to claim 5, wherein said site of mutation is located at a site apart from said overlapping region by 1 to 5 bases.

7. A method for detecting a single nucleotide polymorphism, comprising applying said method according to any one of claims 1 to 6 to a test sample containing said target nucleic acid or a mutant nucleic acid having a single nucleotide polymorphism of said target nucleic acid; and judging from the obtained measurement result whether the nucleic acid in said test sample is said target nucleic acid or said mutant nucleic acid.

8. A method for judging zygosity of a gene, comprising applying said method according to any one of claims 1 to 6 to a test sample containing a gene whose allele comprises a normal gene and an abnormal gene having a single nucleotide polymorphism; and judging from the obtained measurement result the zygosity of said gene.

9. A kit for measuring a nucleic acid, which is used for said method according to any one of claims 1 to 6, comprising at least said labeled probe, said non-labeled probe and said immobilized probe.
